# EUROPÄISCHE PATENTSCHRIFT

(11) **EP 1 916 305 B1**
(45) Veröffentlichungstag und Bekanntmachung des Hinweises auf die Patenterteilung: **30.06.2010**
(21) Anmeldenummer: 07118379.2
(22) Anmeldetag: 12.10.2007
(51) Int. Cl.: C12N 15/70

(54) **DNS-Konstrukt und Verfahren zur fermentativen Herstellung von Fusionsproteinen**
DNA construct and process for the fermentative production of fusion proteins
Construction d'ADN et procédé pour la production par fermentation de protéines de fusion

(30) Priorität: 25.10.2006 DE 102006050332
(43) Veröffentlichungstag der Anmeldung: 30.04.2008
(73) Patentinhaber: Wacker Chemie AG, 81737 München (DE)
(72) Erfinder: Schlösser, Thomas, Dr., 82515, Wolfratshausen (DE); Daßler, Tobias, Dr., 81825, München (DE); Pfeiffer-Schwiesow, Kerstin, 81369, München (DE)
(74) Vertreter: Potten, Holger

(56) Entgegenhaltungen:
- HAGENMAIER SUSANNE ET AL: "A new periplasmic protein of Escherichia coli which is synthesized in spheroplasts but not in intact cells" JOURNAL OF BACTERIOLOGY, Bd. 179, Nr. 6, 1997, Seiten 2073-2076, XP002473028 ISSN: 0021-9193
- PLANSON ANNE-GAELLE ET AL: "Assistance of maltose binding protein to the in vivo folding of the disulfide-rich C-terminal fragment from Plasmodium falciparum merozoite surface protein 1 expressed in Escherichia coli." BIOCHEMISTRY, Bd. 42, Nr. 45, 18. November 2003 (2003-11-18), Seiten 13202-13211, XP002473029 ISSN: 0006-2960
- CORNELIS P: "Expressing genes in different Escherichia coli compartments" CURRENT OPINION IN BIOTECHNOLOGY, LONDON, GB, Bd. 11, Nr. 5, Oktober 2000 (2000-10), Seiten 450-454, XP002343662 ISSN: 0958-1669
- GEORGIOU ET AL: "Preparative expression of secreted proteins in bacteria: status report and future prospects" CURRENT OPINION IN BIOTECHNOLOGY, LONDON, GB, Bd. 16, Nr. 5, Oktober 2005 (2005-10), Seiten 538-545, XP005097199 ISSN: 0958-1669
- ESPOSITO ET AL: "Enhancement of soluble protein expression through the use of fusion tags" CURRENT OPINION IN BIOTECHNOLOGY, LONDON, GB, Bd. 17, Nr. 4, August 2006 (2006-08), Seiten 353-358, XP005592064 ISSN: 0958-1669

### SEQUENCE LISTING

<110> Consortium fuer elektrochemische Industrie GmbH
<120> DNS-Konstrukt und Verfahren zur fermentativen Herstellung von Fusionsproteinen
<130> Co10614
<140>
   <141>
<160> 24
<170> PatentIn Ver. 2.0
<210> 1
   <211> 414
   <212> DNA
   <213> Escherichia coli
<300>
   <303> J. Bacteriol.
   <304> 179
   <305> 6
   <306> 2073-2076
   <307> 1997
<400> 1
   gcagacacca ctaccgcagc accggctgac gcgaagccga tgatgcacca caaaggcaag 60
   ttcggtccgc atcaggacat gatgttcaaa gacctgaacc tgaccgacgc gcagaaacag 120
   cagatccgcg aaatcatgaa aggccagcgt gaccagatga aacgtccgcc gctggaagaa 180
   cgccgcgcaa tgcatgacat cattgccagc gataccttcg ataaagtaaa agctgaagcg 240
   cagatcgcaa aaatggaaga acagcgcaaa gctaacatgc tggcgcacat ggaaacccag 300
   aacaaaattt acaacatcct gacgccggaa cagaaaaagc aatttaatgc taattttgag 360
   aagcgtctga cagaacgtcc agcggcaaaa ggtaaaatgc ctgcaactgc tgaa       414
<210> 2
   <211> 138
   <212> PRT
   <213> Escherichia coli
<300>
   <303> J. Bacteriol.
   <304> 179
   <305> 6
   <306> 2073-2076
   <307> 1997
<400> 2
   Ala Asp Thr Thr Thr Ala Ala Pro Ala Asp Ala Lys Pro Met Met His
   1               5                   10                  15
   His Lys Gly Lys Phe Gly Pro His Gln Asp Met Met Phe Lys Asp Leu
               20                  25                  30
   Asn Leu Thr Asp Ala Gln Lys Gln Gln Ile Arg Glu Ile Met Lys Gly
           35                  40                  45
   Gln Arg Asp Gln Met Lys Arg Pro Pro Leu Glu Glu Arg Arg Ala Met
       50                  55                  60
   His Asp Ile Ile Ala Ser Asp Thr Phe Asp Lys Val Lys Ala Glu Ala
   65                  70                  75                  80
   Gln Ile Ala Lys Met Glu Glu Gln Arg Lys Ala Asn Met Leu Ala His
                   85                  90                  95
   Met Glu Thr Gln Asn Lys Ile Tyr Asn Ile Leu Thr Pro Glu Gln Lys
               100                 105                 110
   Lys Gln Phe Asn Ala Asn Phe Glu Lys Arg Leu Thr Glu Arg Pro Ala
           115                 120                 125
   Ala Lys Gly Lys Met Pro Ala Thr Ala Glu
       130                 135
<210> 3
   <211> 69
   <212> DNA
   <213> Escherichia coli
<300>
   <303> J. Bacteriol.
   <304> 179
   <305> 6
   <306> 2073-2076
   <307> 1997
<400> 3
   atgcgtaaat taactgcact gtttgttgcc tctaccctgg ctcttggcgc ggctaacctg 60
   gcccatgcc 69
<210> 4
   <211> 30
   <212> DNA
   <213> Artificial Sequence
<220>
   <223> Description of Artificial Sequence: Oligonucleotide spy-fw
<400> 4
   ggaattctga aaggaaggat atagaatatg 30
<210> 5
   <211> 28
   <212> DNA
   <213> Artificial Sequence
<220>
   <223> Description of Artificial Sequence: spy-rev
<400> 5
   gctctagatt tacgttagtg gtgatcag 28
<210> 6
   <211> 49
   <212> DNA
   <213> Artificial Sequence
<220>
   <223> Description of Artificial Sequence: T1
<400> 6
   cgaaatcgaa ggtcgttggc aggaatggga acagaaaatc accgctctg 49
<210> 7
   <211> 48
   <212> DNA
   <213> Artificial Sequence
<220>
   <223> Description of Artificial Sequence: T2
<400> 7
   ctggaacagg ctcagatcca gcaggaaaaa aacgaatacg aactgcag 48
<210> 8
   <211> 44
   <212> DNA
   <213> Artificial Sequence
<220>
   <223> Description of Artificial Sequence: T3
<400> 8
   aaactggaca aatgggctag cctgtgggaa tggttctgac tgca 44
<210> 9
<211> 67
<212> DNA
   <213> Artificial Sequence
<220>
   <223> Description of Artificial Sequence: T4
<400> 9
   gtcagaacca ttcccacagg ctagcccatt tgtccagttt ctgcagttcg tattcgtttt 60
   tttcctg 67
<210> 10
   <211> 66
   <212> DNA
   <213> Artificial Sequence
<220>
   <223> Description of Artificial Sequence: T5
<400> 10
   ctggatctga gcctgttcca gcagagcggt gattttctgt tcccattcct gccaacgacc 60
   ttcgat 66
<210> 11
   <211> 52
   <212> DNA
   <213> Artificial Sequence
<220>
   <223> Description of Artificial Sequence: T6
<400> 11
   gatctttttt ccgtccgctt ccgctttcgc ttggcaggaa tgggaacaga aa 52
<210> 12
   <211> 50
   <212> DNA
   <213> Artificial Sequence
<220>
   <223> Description of Artificial Sequence: T7
<400> 12
   atcaccgctc tgctggaaca ggctcagatc cagcaggaaa aaaacgaata 50
<210> 13
   <211> 50
   <212> DNA
   <213> Artificial Sequence
<220>
   <223> Description of Artificial Sequence: T8
<400> 13
   cgaactgcag aaactggaca aatgggctag cctgtgggaa tggttctgat 50
<210> 14
   <211> 76
   <212> DNA
   <213> Artificial Sequence
<220>
   <223> Description of Artificial Sequence: T9
<400> 14
   tctgagcctg ttccagcaga gcggtgattt tctgttccca ttcctgccaa gcgaaagcgg 60
   aagcggacgg aaaaaa 76
<210> 15
   <211> 76
   <212> DNA
   <213> Artificial Sequence
<220>
   <223> Description of Artificial Sequence: T10
<400> 15
   ctagatcaga accattccca caggctagcc catttgtcca gtttctgcag ttcgtattcg 60
   tttttttcct gctgga 76
<210> 16
   <211> 22
   <212> DNA
   <213> Artificial Sequence
<220>
   <223> Description of Artificial Sequence: pJF118-seqfw
<400> 16
   catcggctcg tataatgtgt gg 22
<210> 17
   <211> 44
   <212> DNA
   <213> Artificial Sequence
<220>
   <223> Description of Artificial Sequence: spy-T1249fus
<400> 17
   caacgacctt cgatagtact ttcagcagtt gcaggcattt tacc 44
<210> 18
   <211> 29
   <212> DNA
   <213> Artificial Sequence
<220>
   <223> Description of Artificial Sequence: T1249-fus2
<400> 18
   agtactatcg aaggtcgttg gcaggaatg 29
<210> 19
   <211> 33
   <212> DNA
   <213> Artificial Sequence
<220>
   <223> Description of Artificial Sequence: T1249-BfrI
<400> 19
   tagaccgctt aagtcagaac cattcccaca ggc 33
<210> 20
   <211> 33
   <212> DNA
   <213> Artificial Sequence
<220>
   <223> Description of Artificial Sequence: INFa2b-fw
<400> 20
   actatcgaag gtcgttgtga cttacctcag acc 33
<210> 21
   <211> 32
   <212> DNA
   <213> Artificial Sequence
<220>
   <223> Description of Artificial Sequence: INFa2b-rev
<400> 21 acctcttaag ctattattct ttggaacgca ag 32
   <210> 22
   <211>
   <212> PRT
   <213> Artificial Sequence <220>
<223> Description of Artificial Sequence: reference peptide 1
<400> 22
<210> 23
   <211> 14
   <212> PRT
   <213> Artificial Sequence <220>
<223> Description of Artificial Sequence: reference peptide 2
<400> 23
   Ile Thr Ala Leu Leu Glu Gln Ala Gln Ile Gln Gln Glu Lys
   1 5 10
<210> 24
   <211> 8
   <212> PRT
   <213> Artificial Sequence <220>
<223> Description of Artificial Sequence: reference peptide 3
<400> 24
   Trp Ala Ser Leu Trp Glu Trp Phe
   1 5

## Beschreibung

Die Erfindung betrifft ein DNS-Konstrukt und Verfahren zur fermentativen Herstellung von Fusionsproteinen unter Einsatz des Konstruktes.

Der Markt für rekombinante Proteinpharmazeutika ("Biologics") ist in den letzten Jahren stark gewachsen. Aufgrund der immer noch sehr hohen Produktionskosten für Pharmawirkstoffe auf Proteinbasis, wird laufend nach effizienteren und damit kostengünstigeren Verfahren und Systemen für deren Herstellung gesucht. Als Protein-Produzenten können verschiedene Mikroorganismen, wie Bakterien, Hefen filamentöse Pilze oder auch Pflanzenzellen oder Tierzellen verwendet werden. Aufgrund seiner sehr gut untersuchten Genetik und Physiologie, der kurzen Generationszeit und der einfachen Handhabung, ist allerdings das Gram-negative Enterobakterium *Escherichia coli* (*E. coli*) gegenwärtig der am häufigsten verwendete Organismus zur Produktion rekombinanter Proteine.

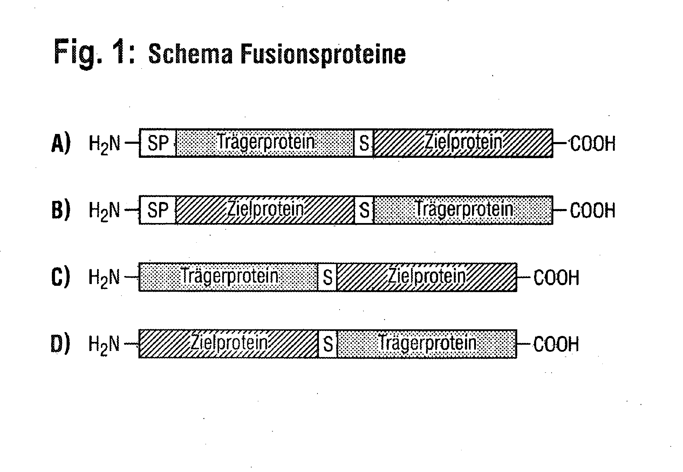

Bei der Herstellung von rekombinanten Proteinen in *E. coli* hat man häufig mit dem Problem der "Inclusion body"-Bildung im Zytoplasma der Zelle zu tun. Diese Schwierigkeit kann oftmals dadurch umgangen werden, indem man das Produktionssystem so auslegt, dass die Zelle das jeweils herzustellende rekombinante Protein, im Folgenden auch als Zielprotein bezeichnet, nicht im Zytoplasma anhäuft, sondern aktiv in das Periplasma und im Idealfall sogar noch weiter bis in das Kulturmedium sekretiert. Dies kann dadurch erreicht werden, dass das Zielprotein von der Zelle zunächst als zytoplamatisches Vorläufer-Fusionsprotein synthetisiert wird (Fig. 1A und 1B), wobei das Signalpeptid (SP) während des Sekretionsvorgangs ins Periplasma *in vivo* abgespalten wird und das Fusionsprotein entsteht (Fig. 1C und 1D). In *E. coli* ist für den Transport und die Abspaltung des Signalpeptids das Sec-System verantwortlich. Prinzipiell werden bei der extrazellulären Herstellung von Zielproteinen Signalpeptide von periplasmatischen oder extrazellulären Proteinen von *E. coli* verwendet. Diese *E. co*li-eigenen Proteine werden über den Sec-Weg sekretiert. Beispielsweise seinen hier die Signalpeptide der *E. coli-*Proteine PhoA, OmpA, STII, Lpp und MalE genannt.

Im Falle des Sec-Systems werden die Proteine im ungefalteten Zustand durch die Zytoplasma-Membran transportiert und anschließend im Periplasma gefaltet. Demgegenüber können mit Hilfe des TAT-Systems (Twin Arginine Translocation) bereits im Zytoplasma gefaltete Proteine in das Periplasma sekretiert werden, wobei aber spezielle Signalpeptide nötig sind (z.B. Signalpeptide der Proteine TorA oder Tap). In dem Übersichtsartikel von Choi und Lee (2004, Appl. Microbiol. Biotechnol. 64, 625-635) ist der Stand der Technik zur sekretorischen und extrazellulären Produktion von rekombinanten Proteinen anhand repräsentativer Beispiele dargestellt.

Die Anhäufung des Zielproteins im Periplasma oder im Kulturmedium hat gegenüber der intrazellulären Produktion die folgenden Vorteile:
u. a.
1) muss der N-terminale Aminosäurerest des sekretierten Zielproteins nicht zwangsläufig Methionin sein, sondern kann identisch zu der natürlichen Startaminosäure des Produkts sein,
2) ist die Protease-Aktivität im Periplasma deutlich geringer als im Zytoplasma,
3) ist die Reinigung eines Proteins aus dem Periplasma bzw. dem Kulturmedium einfacher als aus dem Zytoplasma, da dort weniger verunreinigende Wirtsproteine enthalten sind und
4) wird die. Ausbildung von evtl. notwendigen Disulfidbrücken unter den oxidativen Bedingungen des Periplasmas ermöglicht.

In manchen Fällen, z. B. bei der sekretorischen Herstellung von rekombinanten Proteinen oder Peptiden, die besonders empfindlich gegenüber proteolytischem Abbau sind, oder die zur Bildung von "Inclusion bodies" neigen, ist es sinnvoll bzw. für eine effiziente Produktion sogar unerlässlich, wenn das rekombinante Protein nicht direkt an ein Signalpeptid, sondern an den C-Terminus eines weiteren Proteins, des Trägerproteins, gekoppelt wird (siehe Fig. 1 A: Vorläufer-Fusionsprotein und 1 C: Fusionsprotein). Als Teil eines sekretierbaren Fusionsproteins kann oftmals die Löslichkeit des Zielproteins im Fermentationsmedium erhöht werden. Ebenso kann durch die Kopplung an ein Trägerprotein und den raschen Export des gesamten Vorläufer-Fusionsproteins aus dem Zytoplasma ein proteolyseempfindliches Zielprotein bzw. -peptid vor dem intrazellulären Abbau geschützt werden. Um ein sensitives Genprodukt gegen Abbau zu schützen und damit zu stabilisieren, besteht auch die Möglichkeit, das Trägerprotein an den C-Terminus des Zielproteins zu fusionieren (siehe Fig. 1 B: Vorläufer-Fusionsprotein und 1 D: Fusionsprotein). Ein Beispiel dafür ist das Protein Glutathion S-Transferase, dass sowohl als Trägerprotein als auch als Dimerisierungsdomäne bei der Sekretion eines rekombinaten Protease-Inhibitors als Zielprotein verwendet wird (Tudyka und Skerra, 1997, Protein Science 6, 2180-2187).

Unter einem Zielprotein versteht man die im Folgenden genannten Proteine, Proteinfragmente oder Peptide, die extrazellulär oder periplasmatisch in Ausbeuten größer als 50 mg/l produziert werden sollen.

Unter einem Vorläufer-Fusionsprotein versteht man im Folgenden ein Protein bestehend aus einem Trägerprotein und einem Zielprotein und einem Signalpeptid (SP). Trägerprotein und Zielprotein sind durch eine enzymatisch spaltbare Sequenz S miteinander verbunden. Das Signalpeptid ist für die Translokation des Fusionsproteins über die Zytoplasmamembran hinweg notwendig. Dieses Signalpeptid wird während des Sekretionsprozesses ins Periplasma vom Fusionsprotein abgetrennt, wodurch das Fusionsprotein entsteht.

Unter einem Fusionsprotein versteht man im Folgenden ein Protein bestehend aus einem Trägerprotein und einem Zielprotein. Trägerprotein und Zielprotein sind durch eine enzymatisch spaltbare Sequenz S miteinander verbunden.

Unter einem Trägerprotein versteht man den Teil eines Vorläufer-Fusionsproteins bzw. Fusionsproteins, der zur Stabilisierung des Zielproteins verwendet wird.

Nach Abspaltung des Signalpeptids vom Vorläufer-Fusionsprotein wird das Fusionsprotein im Periplasma oder im Kulturmedium angehäuft. Das Zielprotein ist über eine Aminosäuresequenz, die spaltbare Sequenz S, mit dem Trägerprotein verknüpft (siehe Fig. 1), an der in einem nachfolgenden Schritt das Zielprotein *in vitro* enzymatisch abgespalten und somit freigesetzt werden kann.

Als Trägerprotein ist beispielsweise das *E. coli-*Protein YebF als Fusionspartner (Zhang et al., 2006, Nature Biotechnology 24, 100-104 und WO2006017929A1) zur extrazellulären Produktion von Proteinen beschrieben.

Das YebF-Protein ist mit einer Primärstruktur von 118 Aminosäuren und einem Molekulargewicht von 10,8 kDa das bisher kleinste Trägerprotein das zur heterologen Expression von Proteinen in *E. coli* beschrieben ist. Ein Nachteil bei der Verwendung des YebF-Proteins als Trägerprotein ist, dass das Protein insgesamt drei Cystein-Reste enthält, wovon zwei im reifen Teil des Proteins lokalisiert sind. Diese zwei Cystein-Reste können dann im oxidierenden Milieu des Periplasmas mit dem Zielprotein Disulfidbrücken ausbilden, wodurch es zu einer Fehlfaltung des Zielproteins kommen kann. Dies führt im ungünstigsten Fall zu einem unbrauchbaren Zielprotein. Die Ausbildung falscher Disulfidbrücken ist sowohl vor als auch nach der Abspaltung des Zielproteins vom YebF-Protein möglich.

Ein weiteres Trägerprotein ist das OmpF-Protein von *E. coli.* Jeong und Lee beschreiben die extrazelluläre Produktion eines OmpF-β-Endorphin-Fusionsproteins mit einem Derivat des *E. coli*-Stammes BL21 (DE3) (2002, Appl. Environ. Microbiol. 68, 4979-85). Mit einer Primärstruktur von 362 Aminosäuren und einem daraus resultierenden Molekulargewicht von 36 kDa ist das OmpF-Trägerprotein erheblich größer und schwerer als das Trägerprotein YebF. Dies bedeutet, dass eine *E. coli*-Zelle bei der Produktion eines Zielproteins metabolisch deutlich mehr belastet wird, wenn anstelle von YebF OmpF als Trägerprotein verwendet wird. In der o.g. Veröffentlichung von Jeong und Lee wird dies besonders deutlich: bei einer Produktionsleistung von 5,6 g/l Fusionsprotein erhält man lediglich 0,33 g/l β-Endorphin.

Ein weiteres Trägerprotein ist das C-terminale Fragment des HlyA-Proteins von *E. coli.* Das Hämolysin (HlyA-Protein) aus *E. coli* ist ein porenbildendes Exotoxin mit einem Molekulargewicht von 110 kDa, welches bei pathogenen *E. coli*-Stämmen vorkommt, die beispielsweise Harnwegsinfekte verursachen. Das HlyA-Protein wird über ein eigenes Hämolysin-Transportsystem über die innere und äußere Membran aus der Zelle heraustransportiert. Zur Herstellung der Fusionsproteine wird die C-terminale Domäne von 218 Aminosäuren mit einem Molekulargewicht von 23 kDa des Proteins genutzt. Diese ist für die Translokation über beide Membranen hinweg notwendig.
Humanes Interleukin-6 kann mit Hilfe eines HlyA-Trägerproteins extrazellulär im Kulturüberstand produziert werden. Die Ausbeuten sind mit 70 µg/l jedoch äußerst gering (Li et al., 2002, Gene 25, 437-447). Als Fragment eines toxischen *E. coli-*Proteins ist dieses Fragment zur Herstellung von pharmazeutisch wirksamen Proteinen jedoch ungeeignet.

Ferner ist das eukaryotische Protein Hirudin aus *Hirodo medicinalis* als Trägerprotein zur extrazellulären Produktion von Zielproteinen bekannt. Hirudin ist ein Thrombin-Inhibitor, es bindet mit extrem hoher Affinität (Ki = 20 fM) die Protease Thrombin. Das aus 65 Aminosäuren bestehende Peptid bildet mit seinen 6 Cystein-Resten 3 interne Disulfidbrücken aus. Das Trägerprotein Hirudin wird zur extrazellulären Produktion von Zielproteinen in *E. coli* mit einem bakteriellen Signalpeptid fusioniert. Als Signalpeptide werden beispielsweise Sequenzen der *E. coli-*Proteine PhoA oder OmpC (EP1364029B1) oder auch das Signalpeptid der α-Cyclodextrin-Glycosyltransferase (EP0448093B1) verwendet. Aufgrund seiner biologischen Aktivität als Thrombin-Inhibitor ist Hirudin zur Produktion von Pharmaproteinen ungeeignet, denn im Produktionsprozess eines Zielproteins muss sichergestellt sein, dass keine Verunreinigung mit dem Trägerprotein Hirudin vorliegt. Dies macht den Aufreinigungsprozess des Zielproteins sehr aufwendig und damit teuer.

Eine Aufgabe der vorliegen Erfindung ist die Bereitstellung eines DNS Konstruktes, welches eine kostengünstige Herstellung eines Zielproteins in *E.coli* ermöglicht und dabei nicht mit den beschriebenen Nachteilen des Stands der Technik behaftet ist.

Die Aufgabe wird gelöst durch ein DNS Konstrukt bestehend aus einer Nukleinsäuresequenz, kodierend für ein Signalpeptid, welches funktionell mit einem Gen kodierend für ein Trägerprotein verknüpft ist und dieses über ein Gen kodierend für eine spaltbare Sequenz S, die eine Spaltung des Fusionsproteins enzymatisch durch Proteasen ermöglicht mit einem Gen kodierend für das Zielprotein ausgewählt aus der Gruppe Interferone, Interleukine, Interleukinrezeptoren, Interleukinrezeptor-Antagonisten, Granulocyten-Kolonie-stimulierende Faktoren, Granulocyten-Makrophagen-Kolonie-stimulierende Faktoren, Makrophagen-Kolonie-stimulierende Faktoren, Leukämieinhibitoren, Stammzellenwachstumsfaktoren, Tumornekrosefaktoren, Wachstumshormone, insulinartige Wachstumsfaktoren, Fibroblastenwachstumsfaktoren, von Blutplättchen abstammende Wachstumsfaktoren, transformierende Wachstumsfaktoren, Hepatocyten-Wachstumsfaktoren, knochenmorphogenetische Faktoren, Nervenwachstumsfaktoren, vom Gehirn abstammende neurotrophe Faktoren (BDNF), von Gliazelllinien abstammende neurotrophe Faktoren, Angiogenese-Inhibitoren, Gewebeplasminogenaktivatoren, Blutgerinnungsfaktoren, Trypsin-Inhibitoren, Elastase-Inhibitoren, Immunoglobuline, einzelkettigen Antikörper, Komplementbestandteile, hypoxieinduzierte Stressproteine, Proteinkinasen, Proto-Oncogen-Produkte, Transkriptionsfaktoren, viruskonstitutive Proteine, Proinsulin, Prourokinase, Erythro poietin, Thrombopoietin, Neurotrophin, Protein C, Glucocerebrosidase, Superoxid-Dismutase, Renin, Lysozym, P450, Prochymosin, Lipocortin, Reptin, Serumalbumin, Streptokinase, Tenecteplase und CNTF (ciliary neurotrophic factor) verknüpft ist, **dadurch gekennzeichnet, dass** das Gen kodierend für ein Trägerprotein das spy-Gen aus *E. coli* ist.

Das spy-Gen ist charakterisiert durch SEQ ID No 1 bzw. die durch den degenerierten genetischen Code bedingten analogen Sequenzen, die für das Spy-Protein kodieren.

Das Spy-Protein ist ein periplamatisches Protein kodiert durch SEQ ID NO 2. Diese Sequenz entspricht dem reifen Spy-Protein, so wie es nach der Sekretion in das Periplasma und Abspaltung seiner Signalsequenz vorliegt. Die Bildung des Spy-Protein wird bei der Herstellung von Spheroplasten induziert (Hagenmaier et al., J. Bacteriol. 179, 2073-2076). Auch eine Indol-Behandlung von *E. coli-*Zellen induziert die Spy-Protein-Bildung (Garbe et al., 2000, Arch. Microbiol. 173, 78-82). Im Vergleich zu den Trägerproteinen des Stands der Technik weist das Spy-Protein als Trägerprotein die folgenden Vorteile auf:
a) es ist ein kleines extrazelluläres *E. coli*-Protein;
b) es weist keine Cystein-Reste auf;
c) es stammt aus einem nicht-pathogenen *E. coli-* Stamm
d) es ist ein Trägerprotein aus einem prokaryotischen Organismus ohne Aktivität in einem eukaryotischen Organismus;
e) es ermöglicht eine Produktion eines rekombinanten zielproteins in hohen Ausbeuten unter Beibehaltung seiner Aktivität kostengünstig im Kulturüberstand.

Die Nukleinsäuresequenz des Signalpeptids ist vorzugsweise eine Sequenz kodierend für ein Signalpeptid, die sich zur extrazellulären Herstellung von rekombinanten Proteinen bewährt hat. Bevorzugt handelt es sich um die Signalpeptid-kodierenden Nukleinsäuresequenzen der *E. coli*-Gene ompA, phoA, ompT, lpp, phoE, ompF, lamB, ompC und malE sowie die Signalpeptid-kodierende Nukleinsäuresequenz des spy-Gens aus *E. coli* (SEQ ID No 3). Besonders bevorzugt ist die Signalpeptid-kodierende Nukleinsäuresequenz des spy-Gens aus *E. coli.*

Bevorzugt kodiert die Nukleinsäuresequenz für ein Zielprotein ausgewählt aus der Gruppe der Interferone und Peptide.

Bei der spaltbaren Sequenz S zur kovalenten Verknüpfung von Träger- und Zielprotein handelt es sich vorzugsweise um eine Nukleinsäure-Sequenz, die im translatierten Fusionsprotein die Abspaltung des Zielproteins durch die eukaryotische Protease Faktor Xa ermöglicht.

Zur Herstellung des erfindungsgemäßen DNS-Konstruktes erfolgt die Klonierung der Nukleinsäure-Sequenzen kodierend für das Spy-Protein, das Zielprotein, das Sigalpeptid sowie der spaltbaren Sequenz S zur Freisetzung des Zielproteins in beliebiger zeitlicher Reihenfolge in das Plasmid. Die Orientierung von Zielprotein und Spy-Protein kann dabei, wie in Fig. 1 A und 1 B gezeigt, variiert werden.

Die Klonierung des spy-Gens erfolgt beispielsweise durch spezifische Amplifikation mittels der Polymerase-Ketten-Reaktion (PCR) unter Einsatz von spezifischen Oligonukleotiden, die sich aus SEQ ID No 1 ableiten lassen, wobei die entstandenen PCR-Fragmente durch eine gezielte Ligation in ein Plasmid eingebracht werden können.

Als Plasmid können alle verfügbaren und gentechnisch zugänglichen DNS-Moleküle verwendet werden, die extrachromosomal in Mikroorganismen repliziert werden und die einen Selektionsmarker umfassen. So können beispielsweise Plasmide mit einer hohen zellulären Kopienzahl in *E. coli* (z. B. Plasmide der pUC-Serie, Plasmide der pQE-Serie, Plasmide der pBluescript-Serie), Plasmide mit einer mittleren Kopienzahl in *E. coli* (z. B. Plasmide der pBR-Serie, Plasmide der pACYC-Serie) oder Plasmide mit einer niedrigen Kopienzahl in *E. coli* (z. B. pSC101 oder pBeloBAC11) eingesetzt werden. Bevorzugte verwendet werden Plasmide mit einer hohen zellulären Kopienzahl in *E. coli.* Vorzugsweise wird ein Plasmid eingesetzt, das bereits Elemente zur Herstellung eines Fusionsproteins enthält, wie z.B. eine Nukleinsäure-Sequenz, kodierend für ein Signalpeptid, und eine spaltbare Sequenz S, die die Trennung von Träger- und Zielprotein ermöglicht. Falls diese Elemente zur Herstellung eines Fusionsproteins noch nicht auf dem Plasmid vorhanden sind, werden diese mittels dem Fachmann bekannten Klonierungstechniken in das Plasmid derartig eingebracht, dass die einzelnen Elemente funktionell miteinander verknüpft sind. Anschließend kann das erfindungsgemäße Konstrukt aus dem Plasmid herausgeschnitten werden, beispielsweise mittels geeigneter Restriktionsendonukleasen. Vorzugsweise wird jedoch das Plasmid, enthaltend das erfindungsgemäße DNS-Konstrukt, zur Transformation eines Mikroorganismus eingesetzt.

Die Erfindung betrifft somit auch ein Plasmid enthaltend das erfindungsgemäße DNS-Konstrukt.

Durch eine Transformationsmethode wie z.B. Elektroporation oder die Hitzeschock-Methode werden die erfindungsgemäßen Plasmide in die Zellen eines Mikroorganismenstammes eingebracht. Plasmid-tragende Klone werden anschließend selektiert. Die Selektion erfolgt anhand des auf dem Plasmid befindlichen Selektionsmarkers, der eine Resistenz gegenüber einem bestimmten Antibiotikum vermittelt. Bevorzugt werden Selektionsmarker verwendet, die Resistenz gegenüber Ampicillin oder Tetracyclin vermitteln. Besonders bevorzugt wird ein Selektionsmarker verwendet, der Resistenz gegenüber Tetracyclin vermittelt.

Alternativ zur Klonierung in einem Plasmid kann das erfindungsgemäße DNS-Konstrukt auch in das Chromosom des zur Produktion des rekombinanten Zielproteins verwendeten Mikroorganismenstammes integriert werden. Als Integrationsverfahren werden vorzugsweise die dem Fachmann bekannten Systeme mit temperenten Bakteriophagen, integrativen Plasmiden oder die Integration über homologe Rekombination genutzt.

Die Erfindung betrifft somit auch ein Verfahren zur Herstellung eines Fusionsprotein-sekretierenden Mikroorganismenstammes, **dadurch gekennzeichnet, dass** in einen Mikroorganismenstamm aus der Familie der Enterobacteriaceae ein erfindungsgemäßes DNS-Konstrukt eingebracht wird. Vorzugsweise wird das erfindungsgemäße DNS-Konstrukt dabei in Form eines erfindungsgemäßen Plasmids eingesetzt.

Bevorzugt werden Stämme der Art *Escherichia coli* (z. B. *E. co*li W3110 ATCC 27325) verwendet. Besonders bevorzugt werden *E. coli*-Stämme zur extrazellulären Produktion von rekombinanten Fusionsproteinen eingesetzt, die im Vergleich zu *E. coli* W3110 eine mindestens um den Faktor 2 verbesserte Sekretionsleistung von rekombinanten Fusionsproteinen aufweisen.

Dabei handelt es sich vorzugsweise um folgende *E.* coli-Stämme:
a) BLR: Ray et al. 2002; käuflich erhältlich über Novagen (Merck Biosciences GmbH, 65796 Bad Soden, Deutschland)
b) K802 = CGSC 5610 ; Yang et al., 1998 ; käuflich erhältlich über das *E. coli* Genetic Stock Center CGSC (830 Kline Biology Tower, MCD Biology Department, 266 Whitney Ave., PO box 208103, Yale University, New Haven, USA);
c) WCM105: herstellbar gemäß EP0338410B1;
d) MM28 = CGSC 5892 ; Nagahari et al., 1985 ; käuflich erhältlich über das *E. coli* Genetic Stock Center CGSC (830 Kline Biology Tower, MCD Biology Department, 266 Whitney Ave., PO box 208103, Yale University, New Haven, USA);
e) RV308 = ATCC 31608 ; EP0677109B1 ; käuflich erhältlich über LGC Promochem (Mercatorstr. 51, 46485 Wesel, Deutschland)
f) RR1 : ATCC 31434 ; Nagahari et al., 1985 ; käuflich erhältlich über LGC Promochem (Mercatorstr. 51, 46485 Wesel, Deutschland);
g) KG1005 ompT: herstellbar gemäß Wadensten et al., 1991.

Die Erfindung betrifft ferner nach dem Verfahren hergestellte Mikroorganismenstämme. Diese Stämme der Art Escherichia coli sind **dadurch gekennzeichnet, dass** sie ein erfindungsgemäßes DNS-Konstrukt oder ein erfindungsgemäßes Plasmid enthalten.

Die erfindungsgemäßen Mikroorganismenstämme ermöglichen die extrazelluläre Produktion eines Fusionsproteins bestehend aus dem Spy-Protein, einem der genannten Zielproteine und der genannten spaltbaren Sequenz (S).

Unter einer extrazellulären Produktion ist im Sinne der vorliegenden Erfindung die Anhäufung des rekombinanten Fusionsproteins von ≥ 50 mg/l außerhalb des Zytoplasmas der Mikroorganismenzelle zu verstehen. Bevorzugt handelt es sich um die Anhäufung des rekombinanten Fusionsproteins von ≥ 50 mg/l im Kulturmedium.

Die Erfindung betrifft somit auch ein Verfahren zur fermentativen Produktion eines Fusionsproteins mittels eines Mikroorganismenstammes, **dadurch gekennzeichnet, dass** ein erfindungsgemäßer Mikroorganismenstamm in einem Fermentationsmedium kultiviert wird, wobei ein Fusionsprotein produziert wird und das Fermentationsmedium nach der Fermentation von den Zellen des Mikroorganismenstammes abgetrennt wird.

Die Aufreinigung des Fusionsproteins aus dem Periplasma bzw. dem Kulturmedium kann nach bekannten Verfahren, wie Zentrifugation des Mediums zur Abtrennung der Zellen, gegebenenfalls Aufschluss der Zellen, Filtration, und anschließende chromatographische Reinigung, Komplexierung oder Fällung des Proteins erfolgen.

Bevorzugt sind Verfahren, bei denen nach dem Abtrennen des Fermentationsmediums das zielprotein aus dem Periplasma der Zellen aufgereinigt wird, wobei zunächst das Trägerprotein enzymatisch vom Zielprotein getrennt wird und das Zielprotein anschließend aufgereinigt wird.

Besonders bevorzugt sind Verfahren, bei denen nach dem Abtrennen des Fermentationsmediums das Zielprotein aus dem Fermentationsmedium aufgereinigt werden kann. Dabei wird zunächst das Trägerprotein enzymatisch vom Zielprotein getrennt, wie bereits ausgeführt. Anschließend kann das Zielprotein nach den bekannten Verfahren aufgereinigt werden.

Das erfindungsgemäße Verfahren zur Herstellung eines Fusionsionsproteins ermöglicht eine kostengünstige und effiziente Produktion eines Fusionsionsproteins im Kulturüberstand.

Die Fermentation des Bakterienstammes zur erfindungsgemäßen Produktion von Fusionsproteinen erfolgt vorzugsweise in einem Vollmedium oder Minimalsalzmedium. Diese Medien sind aus der Literatur bekannt.

Als Kohlenstoffquelle können prinzipiell alle verwertbaren Zucker, Zuckeralkohole, organische Säuren bzw. deren Salze, Stärkehydrolysate, Melasse oder andere Stoffe verwendet werden. Dabei werden bevorzugt Glucose oder Glycerin eingesetzt. Auch ist eine kombinierte Fütterung mehrerer verschiedener Kohlenstoffquellen möglich. Als Stickstoffquellen können Harnstoff, Ammoniak und seine Salze, Nitratsalze sowie andere N-Quellen dienen. Zu den möglichen Stickstoffquellen gehören auch komplexe Aminosäuregemische wie Hefeextrakt, Pepton, Malzextrakt, Sojapepton, Casaminosäuren, Maisquellwasser (Corn Steep Liquor) und NZ-Amine (z.B. Kerry Bio-Science, Chicago, USA).

Des weiteren können dem Medium weitere Komponenten zugegeben werden wie Vitamine, Salze, Hefeextrakt, Aminosäuren und Spurenelemente, durch die das Zellwachstum verbessert wird.

Die Inkubation des Stammes erfolgt vorzugsweise unter aeroben Kultivierungsbedingungen über einen Zeitraum von 16 - 150 h und im Bereich der für den jeweiligen Stamm optimalen Wachstumstemperatur.

Als optimaler Temperaturbereich werden 15 - 55 °C bevorzugt. Besonders bevorzugt ist eine Temperatur zwischen 30 und 37 °C.

Die Anzucht des Stammes kann im Schüttelkolben oder Fermenter erfolgen, wobei keine Beschränkungen hinsichtlich Volumen gegeben sind. Die Anzucht kann im Batch-Verfahren, im Fed-Batch- oder in einem kontinuierlichen Verfahren erfolgen.

Die Verwendung des Spy-Proteins als Trägerprotein hat gegenüber der Verwendung der bekannten Trägerproteine die folgenden Vorteile:
a) die Fusionsproteine werden stabilisiert,
b) eine Fehlfaltung des Zielproteins wird verhindert,
c) hohe extrazelluläre Ausbeuten des Zielproteins werden ermöglicht.

Fig. 1 zeigt schematische Darstellungen von
   A) einem vorläufer-Fusionsprotein, bestehend aus einem Signalpeptid (SP) und einem Trägerprotein, an das über eine enzymatisch spaltbare Sequenz (S) das rekombinante Zielprotein gekoppelt ist.
   B) einem Vorläufer-Fusionsprotein, bestehend aus einem Sig- nalpeptid (SP) und dem rekombinanten Zielprotein, an das über eine enzymatisch spaltbare Sequenz (S) ein Träger- protein gekoppelt ist.
   C) einem Fusionsprotein, bestehend aus einem Trägerprotein, an das über eine enzymatisch spaltbare Sequenz (S) das rekombinante Zielprotein gekoppelt ist.
   D) einem Fusionsprotein, bestehend aus einem rekombinanten Zielprotein, an das über eine enzymatisch spaltbare Se- quenz (S) ein Trägerprotein gekoppelt ist.
Fig. 2 zeigt das Plasmid pKP689
Fig. 3 zeigt das Plasmid pT1249
Fig. 4 zeigt das Plasmid pKP700
Fig. 5 zeigt das Plasmid pEX-spy-IFNa2b Fig. 6 zeigt das mittels Coomassie-Färbung gefärbte SDS-Gel aus Bsp. 5

Die folgenden Beispiele dienen der weiteren Erläuterung der Erfindung.

### Beispiel 1: Konstruktion des Plasmids pKP689

### a) Amplifizierung des spy-Gens

Das spy-Gen aus *E. coli* wurde mittels der Polymerase-Ketten-Reaktion (PCR) unter Verwendung der Taq-DNS-Polymerase (Roche, Mannheim) nach gängiger, dem Fachmann bekannter Praxis amplifiziert. Als Matrize diente die chromosomale DNS des *E. coli-*Wildtypstammes W3110 (ATCC 27325). Als Primer wurden die Oligonukleotide spy-fw (SEQ ID No 4) mit der Sequenz 5'-GGA ATT CTG AAA GGA AGG ATA TAG AAT ATG-3' und spy-rev (SEQ ID No 5) mit der Sequenz 5'-GCT CTA GAT TTA CGT TAG TGG TGA TCA G-3' verwendet.

Das bei der PCR erhaltene DNS-Fragment mit einer Länge von ca. 651 Basenpaaren wurde anschließend mittels eines DNS-Adsorptionssäulchens des QIAprep Spin Miniprep Kits (Qiagen, Hilden) nach Herstellerangaben gereinigt.

### b) Klonierung des spy-Gens in den Plasmid-Vektor pEX2200

In das PCR-Fragment wurden über die Primer spy-fw und spy-rev zwei Schnittstellen für die Restriktionsendonukleasen EcoRI und XbaI eingeführt. Das gereinigte PCR-Fragment wurde mit den Restriktionsendonukleasen EcoRI (Roche, Mannheim) und XbaI (Roche, Mannheim) unter den vom Hersteller angegebenen Bedingungen geschnitten, über ein Agarosegel aufgetrennt und dann mittels des QIAquick Gel Extraction Kits (Qiagen, Hilden) nach Herstellerangaben aus dem Agarosegel isoliert.

Zur Klonierung des spy-Gens wurde der Vektor pEX2200 mit den Restriktionsendonukleasen EcoRI und XbaI unter den vom Hersteller (Roche, Mannheim) angegebenen Bedingungen geschnitten. Das Plasmid wurde anschließend durch Behandlung mit Alkalischer Phosphatase (Roche, Mannheim) an den 5'-Enden dephosphoryliert und dann wie das PCR-Fragment mittels QIAquick Gel Extraction Kit (Qiagen, Hilden) gereinigt. Die Ligation des PCR-Fragments mit dem geschnittenen und dephosphorylierten Vektor erfolgte nach Herstellerangaben unter Verwendung der T4-DNS-Ligase (Roche, Mannheim). Die Transformation von *E. coli*-Zellen des Stammes W3110 (ATCC 27325) mit dem Ligationsansatz wurde mittels Elektroporation in einer dem Fachmann bekannten Art und Weise durchgeführt. Der Transformationsansatz wurde auf LB-Tetracyclin-Agarplatten (10 g/l Trypton, 5 g/l Hefeextrakt, 5 g/l NaCl, 15 g/l Agar, 20 mg/l Tetracyclin) ausgebracht und über Nacht bei 37 °C inkubiert.

Die gewünschten Transformanten wurden nach einer Plasmid-isolierung mittels eines QIAprep Spin Miniprep Kit (Qiagen, Hilden) durch eine Restriktionsanalyse identifiziert und die Fehlerfreiheit durch Sequenzanalyse bestätigt.

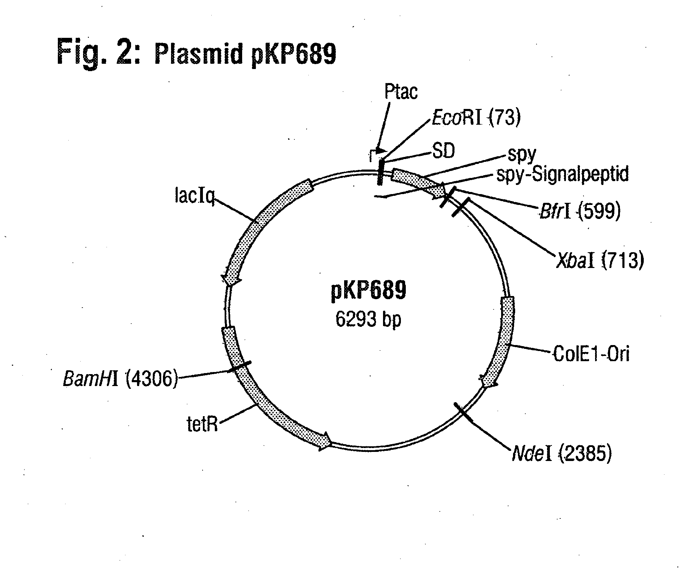

Im dem auf diese Weise erhaltenen Plasmid pKP689 (Fig. 2) ist das spy-Gen unter Kontrolle des induzierbaren tac-Promotors. Das Plasmid pKP689, das für die Ausführung der weiteren Beispiele verwendet wurde, wurde am 26.06.2006 bei der DSMZ (Deutsche Sammlung für Mikroorganismen und Zellkulturen GmbH, D-38142 Braunschweig) unter der Nummer DSM 18381 gemäß Budapester Vertrag hinterlegt.

Das Peptid T1249 ist ein Fusionsinhibitor, der die Fusion von HIV mit der Wirtzellmembran verhindert. Das Peptid ist in seiner aktiven Form am C-terminalen Ende aminiert und am N-terminalen Ende acetyliert. In den Beispielen 2 bis 6 ist die Produktion des unmodifizierten T1249-Peptids beschrieben.

### Beispiel 2: Klonierung von T1249

### a) Vorbereitung des Vektors pCMT203

Der Plasmid-Vektor pCMT203 (herstellbar gemäß EP0448093B1, Beispiel 6) wurde zur Klonierung der T1249-DNS mit den Restriktionsendonukleasen SfuI und PstI unter den vom Hersteller (Roche, Mannheim) angegebenen Bedingungen geschnitten. Das Plasmid wurde anschließend durch Behandlung mit Alkalischer Phosphatase (Roche, Mannheim) an den 5'-Enden dephosphoryliert und dann wie das PCR-Fragment mittels QIAquick Gel Extraction Kit (Qiagen, Hilden) gereinigt.

### b) Phosphorylierung von T1249-DNS-Fragmenten

Die Nukleinsäuresequenz für das Peptid T1249 wurde mit Hilfe von Oligonukleotiden synthetisch hergestellt. Die Oligonukleotide T1 (SEQ ID No 6), T2 (SEQ ID No 7), T3 (SEQ ID No 8), T4 (SEQ ID No 9) und T5 (SEQ ID No 10) wurden mit Hilfe einer Polynukleotid-Kinase (Roche, Mannheim) nach Angaben des Herstellers phosphoryliert.

### c) Ligation

Die Ligation der phosphorylierten T1249-DNS-Fragmente mit dem geschnittenen und dephosphorylierten Vektor erfolgte nach Herstellerangaben unter Verwendung der T4-DNS-Ligase (Roche, Mannheim). Die Transformation von *E. coli-*Zellen des Stammes W3110 (ATCC 27325) mit dem Ligationsansatz wurde mittels Elektroporation in einer dem Fachmann bekannten Art und Weise durchgeführt. Der Transformationsansatz wurde auf LB-Tetracyclin-Agarplatten (10 g/l Trypton, 5 g/l Hefeextrakt, 5 g/l NaCl, 15 g/l Agar, 20 mg/l Tetracyclin) ausgebracht und über Nacht bei 37 °C inkubiert.

Die gewünschten Transformanten wurden nach einer Plasmid-isolierung mittels eines QIAprep Spin Miniprep Kit (Qiagen, Hilden) durch eine Restriktionsanalyse identifiziert und die Fehlerfreiheit durch Sequenzanalyse bestätigt.

Im dem auf diese Weise erhaltenen Plasmid pT1249fus ist die T1249-DNS durch eine durch die Protease Faktor Xa-spaltbare DNS-Sequenz vom Hirudin-Fragment separiert.

### Beispiel 3: Konstruktion von pT1249

### a) Vorbereitung des Vektors pBaBIFN1

Der Plasmid-Vektor pBaBIFN1 (isolierbar aus dem Stamm DH5α/pBaBIFN1 hinterlegt gemäß Budapester Vertrag bei der DSMZ (Deutsche Sammlung für Mikroorganismen und Zellkulturen GmbH, D-38142 Braunschweig unter der Nummer DSM 18343) wurde zur Klonierung der T1249-DNS mit den Restriktionsendonukleasen BglII und XbaI unter den vom Hersteller (Roche, Mannheim) angegebenen Bedingungen geschnitten. Das Plasmid wurde anschließend durch Behandlung mit Alkalischer Phosphatase (Roche, Mannheim) an den 5'-Enden dephosphoryliert und dann wie das PCR-Fragment mittels QIAquick Gel Extraction Kit (Qiagen, Hilden) gereinigt.

### b) Phosphorylierung von T1249-DNS-Fragmenten

Die Nukleinsäuresequenz für das Peptid T1249 wurde mit Hilfe von Oligonukleotiden synthetisch hergestellt. Die Oligonukleotide T6 (SEQ ID No 11), T7 (SEQ ID No 12), T8 (SEQ ID No 13), T9 (SEQ ID No 14) und T10 (SEQ ID No 15) wurden mit Hilfe einer Polynukleotid-Kinase (Roche, Mannheim) nach Angaben des Herstellers phosphoryliert.

### c) Ligation

Die Ligation der phosphorylierten T1249-DNS-Fragmente mit dem geschnittenen und dephosphorylierten Vektor erfolgte nach Herstellerangaben unter Verwendung der T4-DNS-Ligase (Roche, Mannheim). Die Transformation von *E. coli-*Zellen des Stammes W3110 (ATCC 27325) mit dem Ligationsansatz wurde mittels Elektroporation in einer dem Fachmann bekannten Art und Weise durchgeführt. Der Transformationsansatz wurde auf LB-Tetracyclin-Agarplatten (10 g/l Trypton, 5 g/l Hefeextrakt, 5 g/l NaCl, 15 g/l Agar, 20 mg/l Tetracyclin) ausgebracht und über Nacht bei 37 °C inkubiert.

Die gewünschten Transformanten wurden nach einer Plasmid-isolierung mittels eines QIAprep Spin Miniprep Kit (Qiagen, Hilden) durch eine Restriktionsanalyse identifiziert und die Fehlerfreiheit durch Sequenzanalyse bestätigt.

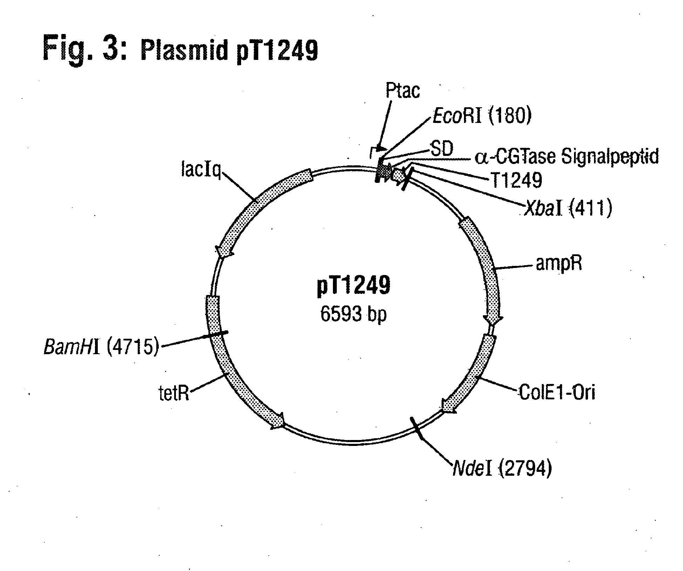

Eine Plasmidkarte von Plasmid pT1249 ist in Fig. 3 gezeigt.

### Beispiel 4: Konstruktion einer spy-T1249-Fusion

### a) Amplifizierung des spy-Gens

Das spy-Gen wurde mittels der Polymerase-Ketten-Reaktion (PCR) unter Verwendung der Taq-DNS-Polymerase (Roche, Mannheim) nach gängiger, dem Fachmann bekannter Praxis amplifiziert. Als Matrize diente das Plasmid pKP689. Als Primer wurden die Oligonukleotide pJF118-seqfw (SEQ ID No 16) mit der Sequenz 5'-CAT CGG CTC GTA TAA TGT GTG G-3' und spy-T1249fus (SEQ ID No 17) mit der Sequenz 5'-CAA CGA CCT TCG ATA GTA CTT TCA GCA GTT GCA GGC ATT TTA CC-3' verwendet.

Das bei der PCR erhaltene DNS-Fragment mit einer Länge von ca. 586 Basenpaaren wurde anschließend mittels eines DNS-Adsorptionssäulchens des QIAprep Spin Miniprep Kits (Qiagen, Hilden) nach Herstellerangaben gereinigt.

### b) Amplifizierung des T1249-Gens

Das T1249-Gen wurde mittels der Polymerase-Ketten-Reaktion (PCR) unter Verwendung der Taq-DNS-Polymerase (Roche, Mannheim) nach gängiger, dem Fachmann bekannter Praxis amplifiziert. Als Matrize diente das Plasmid pT1249fus. Als Primer wurden die Oligonukleotide T1249-fus2 (SEQ ID No 18) mit der Sequenz 5'-AGT ACT ATC GAA GGT CGT TGG CAG GAA TG-3' und T1249-BfrI (SEQ ID No 19) mit der Sequenz 5'-TAG ACC GCT TAA GTC AGA ACC ATT CCC ACA GGC-3' verwendet.

Das bei der PCR erhaltene DNS-Fragment mit einer Länge von ca. 151 Basenpaaren wurde anschließend mittels eines DNS-Adsorptionssäulchens des QIAprep Spin Miniprep Kits (Qiagen, Hilden) nach Herstellerangaben gereinigt.

### c) Fusion von der spy- und T1249-Gene

Die Gene spy und T1249 wurden mittels der Polymerase-Ketten-Reaktion (PCR) unter Verwendung der Taq-DNS-Polymerase (Roche, Mannheim) nach gängiger, dem Fachmann bekannter Praxis amplifiziert. Als Matrizen wurden gleichzeitig die gereinigten PCR-Fragmente aus den Beispielen 4a und 4b (siehe oben) eingesetzt. Als Primer wurden die Oligonukleotide pJF118-seqfw (SEQ ID No 16) mit der Sequenz 5'-CAT CGG CTC GTA TAA TGT GTG G-3' und T1249-BfrI (SEQ ID No 19) mit der Sequenz 5'-TAG ACC GCT TAA GTC AGA ACC ATT CCC ACA GGC-3' verwendet.

Das bei der PCR erhaltene DNS-Fragment mit einer Länge von ca. 717 Basenpaaren wurde anschließend mittels eines DNS-Adsorptionssäulchens des QIAprep Spin Miniprep Kits (Qiagen, Hilden) nach Herstellerangaben gereinigt.

### d) Klonierung der spy-T1249-Fusion in den Plasmid-Vektor pKP689

Das gereinigte PCR-Fragment aus Beispiel 4c (siehe oben) wurde mit den Restriktionsendonukleasen EcoRI (Roche, Mannheim) und BfrI (Roche, Mannheim) unter den vom Hersteller angegebenen Bedingungen geschnitten, über ein Agarosegel aufgetrennt und dann mittels des QIAquick Gel Extraction Kits (Qiagen, Hilden) nach Herstellerangaben aus dem Agarosegel isoliert.

Zur Klonierung der spy-T1249-Fusion wurde der Vektor pKP689 mit den Restriktionsendonukleasen EcoRI und BfrI unter den vom Hersteller (Roche, Mannheim) angegebenen Bedingungen geschnitten. Der größere Teil des Plasmids (ca. 6053 Basenpaare) wurde über ein Agarosegel vom Rest des Plasmids getrennt und dann mittels des QIAquick Gel Extraction Kits (Qiagen, Hilden) nach Herstellerangaben aus dem Agarosegel isoliert.

Das DNS-Fragment wurde anschließend durch Behandlung mit Alkalischer Phosphatase (Roche, Mannheim) an den 5'-Enden dephosphoryliert und dann wie das PCR-Fragment mittels QIAquick Gel Extraction Kit (Qiagen, Hilden) gereinigt. Die Ligation des PCR-Fragments mit dem geschnittenen und dephosphorylierten DNS-Fragment erfolgte nach Herstellerangaben unter Verwendung der T4-DNS-Ligase (Roche, Mannheim). Die Transformation von *E. coli*-Zellen des Stammes W3110 (ATCC 27325) mit dem Ligationsansatz wurde mittels Elektroporation in einer dem Fachmann bekannten Art und Weise durchgeführt. Der Transformationsansatz wurde auf LB-Tetracyclin-Agarplatten (10 g/l Trypton, 5 g/l Hefeextrakt, 5 g/l NaCl, 15 g/l Agar, 20 mg/l Tetracyclin) ausgebracht und über Nacht bei 37 °C inkubiert. Die gewünschten Transformanten wurden nach einer Plasmid-isolierung mittels eines QIAprep Spin Miniprep Kit (Qiagen, Hilden) durch eine Restriktionsanalyse identifiziert und die Fehlerfreiheit durch Sequenzanalyse bestätigt.

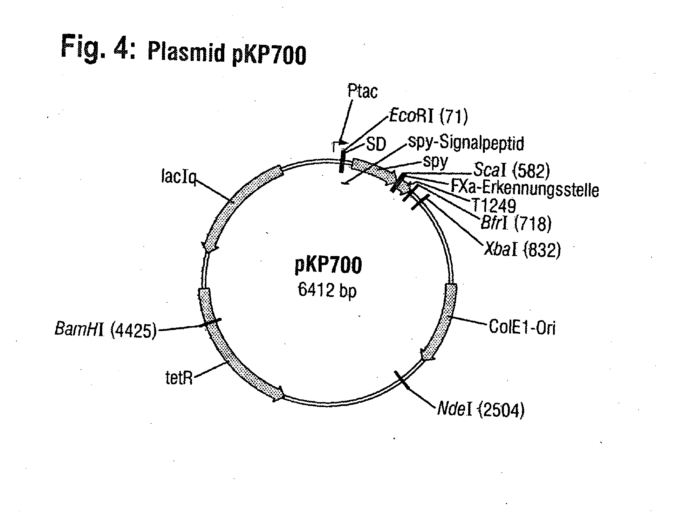

Im dem auf diese Weise erhaltenen Plasmid pKP700 (Fig. 4) ist die spy-T1249-Fusion unter Kontrolle des induzierbaren tac-Promotors.

### Beispiel 5: Sekretion des unmodifizierten T1249-Peptids in den Kulturüberstand

Die Sekretion des Spy-T1249-Fusionsproteins in den Kulturüberstand wurde mit der Sekretion des unmodifizierten T1249-Peptids in den Kulturüberstand verglichen. Dazu wurden die beiden Stämme WCM105/pKP700 und WCM105/pT1249 in 10 ml LB-Medium in einem 100 ml Erlenmeyerkolben mit 10 g/l Glucose bei 30°C angezogen. Der *E. coli-*Stamm WCM105 ist herstellbar gemäß EP0338410B1.

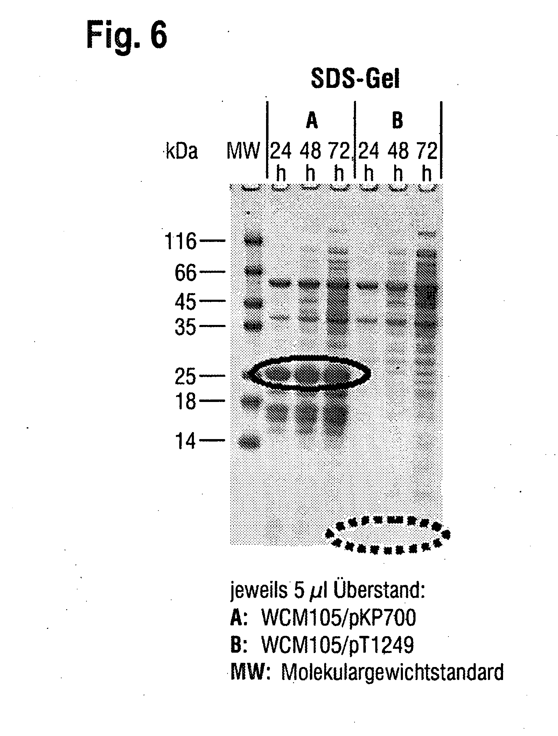

Bei OD₆₀₀ = 0,5 wurde die Produktion von T1249 bzw. des Fusionsproteins durch Zugabe von Isopropylthiogalactosid (0,5 mM) induziert. Im Überstand der Anzuchten wurde nach 24 h, 48 h und 72 h die Menge an gebildetem und sekretierten T1249 bzw. Fusionsprotein durch Auftrennung der Proteine bzw. Peptide im SDS-Gel und Detektion mittels Coomassie-Färbung untersucht. Das Ergebnis ist in Fig. 6 dargestellt. Deutlich zu erkennen ist, dass das Spy-T1249-Fusionsprotein im Kulturüberstand angehäuft wird (siehe ovale Markierung im Gel). Das Spy-Peptid häuft sich nicht im Kulturüberstand an (siehe gestrichelte ovale Markierung im Gel).

### Beispiel 6: Produktion des unmodifizierten T1249-Peptids

Die Produktion des unmodifizierten T1249-Peptids wurde in den Stämmen WCM105/pT1249 und WCM105/pKP700 untersucht. Dazu wurden die Stämme in 10 ml LB-Medium in einem 100 ml Erlenmeyerkolben mit 10 g/l Glucose bei 30°C angezogen. Bei OD₆₀₀ = 0,5 wurde die Produktion des unmodifizierten T1249-Peptids durch Zugabe von Isopropylthiogalactosid (0,5 mM) induziert.

Im Überstand der Anzuchten wurde nach 24 h, 48 h und 72 h die Menge an gebildetem, unmodifizierten T1249-Peptid mit der AQUA-Methode (Gerber et al., 2003, PNAS 100, 6940-6945; Hochleitner et al., 2005, J. Biol. Chem. 280, 2536-2542) nachgewiesen.

Es wurden drei Referenzpeptide (SEQ ID No 22, SEQ ID No 23 und SEQ ID No 24) aus der Sequenz des unmodifizierten T1249-Peptids ausgewählt, die tryptischen Spaltprodukten dieses Peptids entsprechen. Diese Peptide wurden synthetisiert und die Absolutmenge wurde mittels Abwiegen bestimmt. Danach wurden die Referenzpeptide mit der deuterierten Version des Acetyl-N-oxysuccinimids (D₃-acetyl-N-oxysuccinimid) derivatisiert.

Der Fermenterüberstand wurde mit 6M Guanidiniumhydrochlorid denaturiert, reduziert (DTT) und alkyliert (Jodacetamid) und mit Trypsin verdaut. Der Verdau wurde mit Acetyl-N-oxysuccinimid derivatisiert. Definierte Mengen der Referenzpeptide wurden zugesetzt und der Verdau wurde dann mittels HPLC aufgetrennt. Gesammelte Fraktionen wurden mittels Massenspektrometrie untersucht. Es wurden Isotopenpaare der Referenzpeptide und der Peptide, die aus dem Verdau stammten, beobachtet. Diese Isotopenpaare haben einen Massenunterschied von 3 Dalton. Aus dem Intensitätsverhältnis des Peptids aus dem Verdau und dem Referenzpeptid konnte die Absolutmenge des T1249 Peptids bestimmt werden.

Bei Verwendung des Stammes WCM105/pKP700 wurde vor dem Nachweis über die AQUA-Methode ein Faktor Xa-Verdau durchgeführt um das unmodifizierte T1249-Peptid vom Trägerprotein zu trennen. Ein 50 µl-Ansatz bestand aus 10 µl 5 x Puffer (250 mM Tris, pH 8,0; 0,5 M NaCl, 5 mM CaCl₂), 15 µl Kulturüberstand, 1 µl Faktor Xa (5 µg/µl; Sigma, Taufkirchen, Katalog-Nr. F9302) und 24 µl H₂O. Der Faktor Xa-Verdau erfolgte für 16 h bei 22 °C.

In Tabelle 1 sind die quantifizierten Ausbeuten an unmodifiziertem T1249-Peptid aufgelistet.

**Tabelle 1:**

| Stamm | unmodifiziertes T1249-Peptid [mg/l] | | |
|---|---|---|---|
| | 24 h | 48 h | 72 h |
| WCM105 | 0 | 0 | 0 |
| WCM105/pT1249 | 0 | 21 | 50 |
| WCM105/pKP700 | 4 | 46 | 153 |

### Beispiel 7: Konstruktion einer spy-Interferon α2b-Fusion

### a) Amplifizierung des Interferon α2b-Gens

Das Interferon α2b-Gen wurde mittels der Polymerase-Ketten-Reaktion (PCR) unter Verwendung der Taq-DNS-Polymerase (Roche, Mannheim) nach gängiger, dem Fachmann bekannter Praxis amplifiziert. Als Matrize diente das Plasmid pBaBIFN1 (isolierbar aus DSM 18343). Als Primer wurden die Oligonukleotide IFNa2b-fw (SEQ ID No 20) mit der Sequenz 5'-P^{[1]}-ACT ATC GAA GGT CGT TGT GAC TTA CCT CAG ACC-3' (^{[1]} Oligonukleotid ist am 5'-Ende phosphoryliert) und IFNa2b-rev (SEQ ID No 21) mit der Sequenz 5'-ACC TCT TAA GCT ATT ATT CTT TGG AAC GCA AG-3' verwendet.

Das bei der PCR erhaltene DNS-Fragment mit einer Länge von ca. 526 Basenpaaren wurde anschließend mittels eines DNS-Adsorptionssäulchens des QIAprep Spin Miniprep Kits (Qiagen, Hilden) nach Herstellerangaben gereinigt.

### b) Klonierung des Interferon α2b-Gens in den Plasmid-Vektor pKP700

In das PCR-Fragment wurde über den Primer IFNa2b-rev eine endständige Schnittstelle für die Restriktionsendonuklease BfrI eingeführt. Das gereinigte PCR-Fragment wurde mit der Restriktionsendonuklease BfrI (Roche, Mannheim) unter den vom Hersteller angegebenen Bedingungen geschnitten, über ein Agarosegel aufgetrennt und dann mittels des QIAquick Gel Extraction Kits (Qiagen, Hilden) nach Herstellerangaben aus dem Agarosegel isoliert.

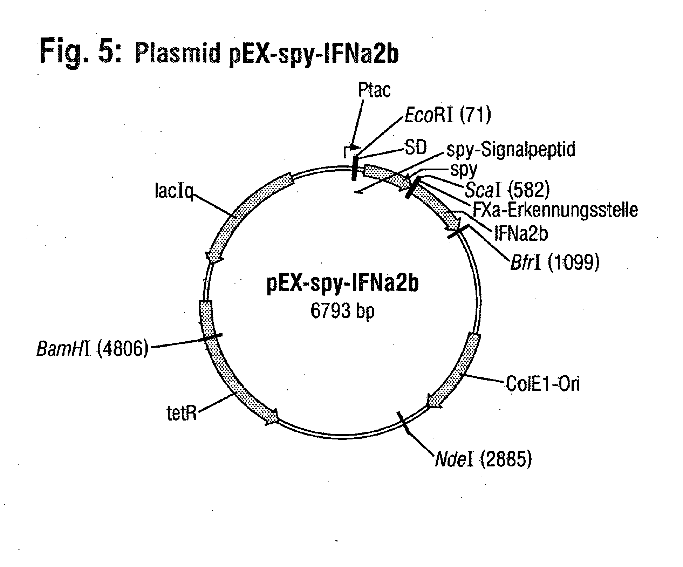

Zur Klonierung des spy-Gens wurde der Vektor pKP700 mit den Restriktionsendonukleasen ScaI und BfrI unter den vom Hersteller (Roche, Mannheim) angegebenen Bedingungen geschnitten. Das Plasmid wurde anschließend durch Behandlung mit Alkalischer Phosphatase (Roche, Mannheim) an den 5'-Enden dephosphoryliert und dann wie das PCR-Fragment mittels QIAquick Gel Extraction Kit (Qiagen, Hilden) gereinigt. Die Ligation des PCR-Fragments mit dem geschnittenen und dephosphorylierten Vektor erfolgte nach Herstellerangaben unter Verwendung der T4-DNS-Ligase (Roche, Mannheim). Die Transformation von *E. coli-*Zellen des Stammes W3110 (ATCC 27325) mit dem Ligationsansatz wurde mittels Elektroporation in einer dem Fachmann bekannten Art und Weise durchgeführt. Der Transformationsansatz wurde auf LB-Tetracyclin-Agarplatten (10 g/l Trypton, 5 g/l Hefeextrakt, 5 g/l NaCl, 15 g/l Agar, 20 mg/l Tetracyclin) ausgebracht und über Nacht bei 37 °C inkubiert. Die gewünschten Transformanten wurden nach einer Plasmid-isolierung mittels eines QIAprep Spin Miniprep Kit (Qiagen, Hilden) durch eine Restriktionsanalyse identifiziert und die Fehlerfreiheit durch Sequenzanalyse bestätigt. Im dem auf diese Weise erhaltenen Plasmid pEX-spy-IFNa2b (Fig. 5) ist das Interferon α2b-Gen unter Kontrolle des induzierbaren tac-Promotors.

### Beispiel 8: Produktion von Interferon α2b

Die Produktion von Interferon α2b in den erhaltenen Stämmen wurde untersucht. Dazu wurden die Stämme in 10 ml LB-Medium in einem 100 ml Erlenmeyerkolben mit 10 g/l Glucose bei 30°C angezogen. Bei OD₆₀₀ = 0,5 wird die Produktion von Interferon α2b durch Zugabe von Isopropylthiogalactosid (0,5 mM) induziert. Im Überstand der Anzuchten wurde nach 24 h, 48 h und 72 h die Menge an gebildetem und sekretierten Interferon α2b durch Auftrennung der Proteine im SDS-Gel und Detektion im Immunoblot mit anti-Interferon-spezifischen Antikörpern nachgewiesen.

Bei Verwendung des Stammes WCM105/pEX-spy-IFNa2b wurde vor dem SDS-Gel ein Faktor Xa-Verdau durchgeführt um das Interferon α2b vom Trägerprotein zu trennen. Ein 50 µl-Ansatz bestand aus 10 µl 5 x Puffer (250 mM Tris, pH 8,0; 0,5 M NaCl, 5 mM CaCl₂), 15 µl Kulturüberstand, 1 µl Fakor Xa (5 µg/µl; Sigma, Taufkirchen, Katalog-Nr. F9302) und 24 µl H₂O. Der Faktor Xa-Verdau erfolgte für 16 h bei 22 °C.

Je 5 µl (Stamm WCM105/pBaBIFN1) bzw. 16,5 µl (Stamm WCM105/pEX-spy-IFNa2b) Überstand wurde mit Probenpuffer versetzt (2 x Tris SDS-Sample-Buffer (Invitrogen, Karlsruhe): 0,125 M Tris-HCl (pH 6,8); 4% w/v SDS; 20% v/v Glycerin; 0,005% v/v Bromphenolblau; 5% v/v β-Mercaptoethanol). Außerdem wurden definierte Mengen von Interferon α2b als Standard mit aufgetragen. Denaturieren der Proteine erfolgte durch Erhitzen auf 100°C für 5 min, Abkühlen für 2 min auf Eis mit abschließendem Abzentrifugieren. Die Proteine wurden durch Elektrophorese in einem 12% NuPAGE^{®} Bis-Tris-Gel (Invitrogen, Karlsruhe) mit 1 x MES-haltigem Running Buffer (Invitrogen, Karlsruhe) aufgetrennt (Elektrophorese-Parameter: 40 min bei 200 V).

Die Detektion und Quantifizierung über einen Immunoblot wurde nach folgender Vorschrift durchgeführt:

### a) Transfer im Nassblot-Verfahren

Modul: Amersham: Hoefer TE 22 Mini Tank Transfer Unit, Code
Number: 80-6204-26.
Membran: Nitrozellulose-Membran (Schleicher & Schuell; BA 85; Zellulosenitrat (E); 0,45 µm Porengröße).

Whatman-Filter und Nitrozellulose-Membran wurden in passende Größen geschnitten und mit Schaumstoffstücken (Schwämmen) in Transferpuffer (Invitrogen, Karlsruhe) luftblasenfrei getränkt.

Aufbau des Sandwiches: schwarzes Gitter, Verbindung mit der Kathode, 2 Schwämme mit je 3 mm Dicke, Whatman-Papier, SDS-Polyacrylamidgel, Nitrozellulose-Membran, Whatman-Papier, 1 Schwamm mit 6 mm Dicke, weißes Gitter, Verbindung mit der Anode.

Transferbedingungen: I = 200 mA konstante Stromstärke, U = unbegrenzt, Laufzeit = 60 min.

### b) Prehybridisierung

### Inkubation der Membran in 25 ml Prehybridisierungspuffer;

30 min bei 20 °C schwenken.

### c) Hybridisierung 1. Antikörper

Inkubation der Membran in 25 ml Prehybridisierungspuffer mit 0,15 µg/ml (-> 3,75 µg) Anti-human-IFN-alpha Antikörper (Pepro Tech EC über Biozol, Eching; Katalognummer 500-P32A); 90 min oder über Nacht bei 20 °C schwenken

### d) Waschen

10 Sekunden mit 1 x PBS bei 20 °C schwenken; Puffer abgießen; 2 x 15 min mit 1 x PBS bei 20 °C schwenken; Puffer abgießen.

### e) Hybridisierung 2. Antikörper

Inkubation der Membran in 25 ml Prehybridisierungspuffer mit 25 µl (1:1000-Verdünnung) Goat Anti-Rabbit IgG Horseradish Peroxidase Conjugate (HRP) (Southern Biotech über Biozol, Eching; Katalognummer 4050-05); 60 min bei 20 °C schwenken f) Waschen
10 Sekunden mit 1 x PBS bei 20 °C schwenken; Puffer abgießen; 2 x 15 min mit 1 x PBS bei 20 °C schwenken; Puffer abgießen.

### g) Detektion über Chemolumineszenz

Lumi-Light Western Blotting Substrate (Roche, Mannheim) vorbereiten: Lumi-Light Luminol / Enhancer Solution und Lumi-Light Stable Peroxide Solution im Verhältnis 1:1 mischen: 3 ml pro Nitrozellulose-Membran.

Blot 5 min bei 20 °C mit Lumi-Light Western Blotting Substrate inkubieren, Überschuss ablaufen lassen, Membran mit Frischhaltefolie bedecken und sofort einen X-Ray Film (Kodak, X-OMAT) auflegen, 2 min exponieren, entwickeln und fixieren. Bei schwachen Signalen wird die Exposition über einen längeren Zeitraum wiederholt.

### h) Puffer

Prehybridisierungspuffer: 5 % w/v Magermilchpulver in 1 x PBS;
10 x PBS: 100 mM NaH₂PO₄; 1,5 M NaCl, pH 7,5 mit NaOH, 0,5 % Triton 100;
1 x PBS: 10 x PBS mit vollentsalztem Wasser 1:10 verdünnen.

### i) Quantifizierung

Eine quantitative Auswertung erfolgte nach Einscannen mit Biorad GS-800 Calibrated Densitometer mittels der Quantity One 1-D-Analysis Software (Biorad, München) durch Vergleich mit dem aufgetragenen Standard.

In Tabelle 2 sind die quantifizierten Ausbeuten an Interferon α2b aufgelistet.

**Tabelle 2:**

| Stamm | Interferon α2b [mg/l] | | |
|---|---|---|---|
| | 24 h | 48 h | 72 h |
| WCM105 | 0 | 0 | 0 |
| WCM105/pBaBIFN1 | 5 | 25 | 145 |
| WCM105/pEX-spy-IFNa2b | 21 | 102 | 410 |

## Patentansprüche

1. DNS Konstrukt, welches eine kostengünstige Herstellung eines Zielproteins in *E.coli* ermöglicht bestehend aus einer Nukleinsäuresequenz kodierend für ein Signalpeptid, welches funktionell mit einem Gen kodierend für ein Trägerprotein verknüpft ist und dieses über ein Gen kodierend für eine spaltbare Sequenz S, die eine Spaltung des Fusionsproteins enzymatisch durch Proteasen ermöglicht mit einem Gen kodierend für das Zielprotein ausgewählt aus der Gruppe Interferone, Interleukine, Interleukinrezeptoren, Interleukinrezeptor-Antagonisten, Granulocyten-Kolonie-stimulierende Faktoren, Granulocyten-Makrophagen-Kolonie-stimulierende Faktoren, Makrophagen-Kolonie-stimulierende Faktoren, Leukämieinhibitoren, Stammzellenwachstumsfaktoren, Tumornekrosefaktoren, Wachstumshormone, insulinartige Wachstumsfaktoren, Fibroblastenwachstumsfaktoren, von Blutplättchen abstammende Wachstumsfaktoren, transformierende Wachstumsfaktoren, Hepatocyten-Wachstumsfaktoren, knochenmorphogenetische Faktoren, Nervenwachstumsfaktoren, vom Gehirn abstammende neurotrophe Faktoren (BDNF), von Gliazelllinien abstammende neurotrophe Faktoren, Angiogenese-Inhibitoren, Gewebeplasminogenaktivatoren, Blutgerinnungsfaktoren, Trypsin-Inhibitoren, Elastase-Inhibitoren, Immunoglobuline, einzelkettigen Antikörper, Komplementbestandteile, hypoxieinduzierte Stressproteine, Proteinkinasen, Proto-Oncogen-Produkte, Transkriptionsfaktoren, viruskonstitutive Proteine, Proinsulin, Prourokinase, Erythropoietin, Thrombopoietin, Neurotrophin, Protein C, Glucocerebrosidase, Superoxid-Dismutase, Renin, Lysozym, P450, Prochymosin, Lipocortin, Reptin, Serumalbumin, Streptokinase, Tenecteplase und CNTF (ciliary neurotrophic factor) verknüpft ist, **dadurch gekennzeichnet, dass** das Gen kodierend für ein Trägerprotein das spy Gen aus *E. coli* ist.

2. DNS Konstrukt gemäß Anspruch 1 **dadurch gekennzeichnet, dass** das spy-Gen die SEQ ID NO 1 bzw. eine durch den degenerierten genetischen Code bedingte analoge Sequenz kodierend für das Spy-Protein aufweist.

3. DNS Konstrukt gemäß Anspruch 1 oder 2 **dadurch gekennzeichnet, dass** die Nukleinsäuresequenz kodierend für ein Signalpeptid ausgewählt ist aus der Gruppe der Signalpeptid-kodierenden Nukleinsäuresequenzen der *E. coli*-Gene ompA, phoA, ompT, lpp, phoE, ompF, lamB, ompC, malE, spy.

4. DNS Konstrukt gemäß Anspruch 1 **dadurch gekennzeichnet, dass** die spaltbare Sequenz S eine Abspaltung des Zielproteins durch die eukaryotische Protease Faktor Xa ermöglicht.

5. Plasmid enthaltend ein DNS-Konstrukt gemäß einem der Ansprüche 1 bis 4.

6. Verfahren zur Herstellung eines Fusionsprotein-sekretierenden Mikroorganismenstammes, **dadurch gekennzeichnet, dass** in einen Mikroorganismenstamm aus der Familie der Enterobacteriaceae ein DNS-Konstrukt gemäß einem der Ansprüche 1 bis 4 oder ein Plasmid gemäß Anspruch 5 eingebracht wird.

7. Mikroorganismenstammder Art *Escherichia coli* enthaltend ein DNS-Konstrukt gemäß einem der Ansprüche 1 bis 4 oder ein Plasmid gemäß Anspruch 5.

8. Verfahren zur fermentativen Produktion eines Fusionsproteins mittels eines Mikroorganismenstammes, **dadurch gekennzeichnet, dass** ein Mikroorganismenstamm gemäß Anspruch 7 in einem Fermentationsmedium kultiviert wird, wobei ein Fusionsprotein gebildet wird und das Fermentationsmedium nach der Fermentation von den Zellen des Mikroorganismenstammes abgetrennt wird.

9. Verfahren, gemäß Anspruch 8 **dadurch gekennzeichnet, dass** nach dem Abtrennen des Fermentationsmediums das Zielprotein aus dem Periplasma der Zellen aufgereinigt wird, wobei zunächst das Trägerprotein enzymatisch vom Zielprotein getrennt wird und das Zielprotein anschließend aufgereinigt wird.

## Claims

1. DNA construct which makes possible the inexpensive production of a target protein in *E. coli,* consisting of a nucleic acid sequence encoding a signal peptide which is operably linked with a gene coding for a carrier protein which is linked with a gene coding for the target protein selected from the group consisting of interferons, interleukins, interleukin receptors, interleukin receptor antagonists, granulocyte-colony-stimulating factors, granulocyte-macrophage-colony-stimulating factors, macrophage-colony-stimulating factors, leukemia inhibitors, stem-cell growth factors, tumor necrosis factors, growth hormones, insulin-like growth factors, fibroblast growth factors, platelet-derived growth factors, transforming growth factors, hepatocyte growth factors, bone-morphogenetic factors, nerve growth factors, brain-derived neurotrophic factors (BDNF), glia-cell-line-derived neurotrophic factors, angiogenesis inhibitors, tissue plasminogen activators, blood clotting factors, trypsin inhibitors, elastase inhibitors, immunoglobulins, single-chain antibodies, complement components, hypoxia-inducing stress proteins, protein kinases, proto-oncogen products, transcription factors, virus-constitutive proteins, proinsulin, prourokinase, erythropoietin, thrombopoietin, neurotrophin, protein C, glucocerebrosidase, superoxide dismutase, renin, lysozyme, P450, prochymosin, lipocortin, reptin, serum albumin, streptokinase, tenecteplase and CNTF (ciliary neurotrophic factor), via a gene coding for a cleavable sequence S which makes possible the cleavage of the fusion protein enzymatically by means of proteases, **characterized in that** the gene coding for a carrier protein is the spy gene from *E. coli.*

2. DNA construct according to Claim 1, **characterized in that** the spy gene has the SEQ ID NO 1 or an analogous sequence which is the result of the degeneracy of the genetic code and which codes for the spy protein.

3. DNA construct according to Claim 1 or 2, **characterized in that** the nucleic acid sequence coding for a signal peptide is selected from the group of the signal-peptide-encoding nucleic acid sequences of the *E. coli* genes ompA, phoA, ompT, lpp, phoE, ompF, lamB, ompC, malE, spy.

4. DNA construct according to Claim 1, **characterized in that** the cleavable sequence S makes possible a cleavage of the target protein by means of the eukaryotic protease factor Xa.

5. Plasmid comprising a DNA construct according to any of Claims 1 to 4.

6. Method of generating a fusion-protein-secreting microorganism strain, **characterized in that** a DNA construct according to any of Claims 1 to 4 or a plasmid according to Claim 5 is introduced into a microorganism strain from the family Enterobacteriaceae.

7. Microorganism strain of the species *Escherichia coli* comprising a DNA construct according to any of Claims 1 to 4 or a plasmid according to Claim 5.

8. Process for the fermentative production of a fusion protein by means of a microorganism strain, **characterized in that** a microorganism strain according to Claim 7 is cultured in a fermentation medium, during which process a fusion protein is formed, and, after the fermentation, the fermentation medium is separated from the cells of the microorganism strain.

9. Process according to Claim 8, **characterized in that,** after the fermentation medium has been separated off, the target protein is purified from the periplasm of the cells, the carrier protein first being removed enzymatically from the target protein and the target protein subsequently being purified.

## Revendications

1. Construction d'ADN qui autorise une préparation économique d'une protéine cible dans *E. coli,* constituée d'une séquence d'acides nucléiques codant pour un peptide signal, qui est lié d'une manière fonctionnelle à un gène codant pour une protéine support, cette dernière étant liée par l'intermédiaire d'un gène codant pour une séquence clivable S, qui autorise un clivage de la protéine de fusion par voie enzymatique grâce à des protéases, à un gène codant pour la protéine cible choisie dans le groupe consistant en les interférons, les interleukines, les récepteurs des interleukines, les antagonistes des récepteurs des interleukines, les facteurs de stimulation des colonies de granulocytes, les facteurs de stimulation des colonies de granulocytes-macrophages, les facteurs de stimulation des colonies de macrophages, les inhibiteurs des cellules leucémiques, les facteurs de croissance des cellules souches, les facteurs de nécrose tumorale, les hormones de croissance, les facteurs de croissance insulino-mimétiques, les facteurs de croissance des fibroblastes, les facteurs de croissance dérivant des plaquettes sanguines, les facteurs de croissance transformants, les facteurs de croissance des hépatocytes, les facteurs morphogénétiques osseux, les facteurs de croissance des nerfs, les facteurs neurotrophiques dérivés du cerveau (BDNF), les facteurs neurotrophiques dérivés des lignées de cellules gliales, les inhibiteurs de l'angiogenèse, les activateurs tissulaires du plasminogène, les facteurs de coagulation sanguine, les inhibiteurs de la trypsine, les inhibiteurs de l'élastase, les immunoglobulines, les anticorps monocaténaires, les constituants du complément, les protéines de choc thermique induit par une hypoxie, les protéinekinases, les produits proto-oncogènes, les facteurs de transcription, les protéines constitutives des virus, la proinsuline, la pro-urokinase, l'érythropoiétine, la thrombopoïétine, la neurotrophine, la protéine C, la glucocérébrosidase, la superoxyde-dismutase, la rénine, le lysozyme, le P450, la prochymosine, la lipocortine, la reptine, la sérumalbumine, la streptokinase, la ténecteplase et le CNTF (facteur neurotrophique ciliaire), **caractérisée en ce que** le gène codant pour une protéine support est le gène spy de *E. coli.*

2. Construction d'ADN selon la revendication 1, **caractérisée en ce que** le gène spy présente la séquence SEQ ID N°1, ou une séquence analogue, définie par le code génétique dégénéré, codant pour la protéine spy.

3. Construction d'ADN selon la revendication 1 ou 2, **caractérisée en ce que** la séquence d'acides nucléiques codant pour un peptide signal est choisie dans le groupe des séquences d'acides nucléiques codant pour un peptide signal des gènes de *E. coli* ompA, phoA, ompT, lpp, phoE, ompF, lamB, ompC, malE, spy.

4. Construction d'ADN selon la revendication 1, **caractérisée en ce que** la séquence clivable S autorise un clivage de la protéine cible par la protéase eucaryote Facteur Xa.

5. Plasmide contenant une construction d'ADN selon l'une des revendications 1 à 4.

6. Procédé de préparation d'une souche de microorganismes secrétant une protéine de fusion, **caractérisé en ce qu'**on introduit dans une souche de microorganismes de la famille des entérobactéries une construction d'ADN selon l'une des revendications 1 à 4 ou un plasmide selon la revendication 5.

7. Souche de microorganismes de l'espèce *Escherichia coli,* contenant une construction d'ADN selon l'une des revendications 1 à 4 ou un plasmide selon la revendication 5.

8. Procédé de production fermentative d'une protéine de fusion à l'aide d'une souche de microorganismes, **caractérisé en ce qu'**on cultive une souche de microorganismes selon la revendication 7 dans un milieu de fermentation, ce qui forme une protéine de fusion, et, après la fermentation, on sépare le milieu de fermentation des cellules de la souche de microorganismes.

9. Procédé selon la revendication 8, **caractérisé en ce que,** après séparation du milieu de fermentation, on purifie la protéine cible à partir du périplasme des cellules, ce pour quoi on sépare d'abord par voie enzymatique la protéine support de la protéine cible, puis on purifie la protéine cible.
